# EUROPÄISCHE PATENTANMELDUNG

(11) **EP 1 785 161 A1**
(43) Veröffentlichungstag der Anmeldung: **16.05.2007**
(21) Anmeldenummer: 05024743.6
(22) Anmeldetag: 11.11.2005
(51) Int. Cl.: A61N 5/10

(54) **Behandlungsraum einer Partikeltherapieanlage, Therapieplan, Verfahren zur Erstellung eines Therapieplans und Bestrahlungsverfahren**

(71) Anmelder: SIEMENS AKTIENGESELLSCHAFT, 80333 München (DE)
(72) Erfinder: Grözinger, Sven Oliver Dr., 91074 Herzogenaurach (DE); Herrmann, Klaus, 90403 Nürnberg (DE); Rietzel, Eike Dr., 64289 Darmstadt (DE); Sommer, Andreas, 91094 Langensendelbach (DE); Zeuner, Torsten Dr., 91052 Erlangen (DE)

(57) **Zusammenfassung**

Die Erfindung betrifft einen Behandlungsraum für eine Partikeltherapieanlage, welches ein während einer Behandlung variabel einstellbares Behandlungsraum-Isozentrum, das einen Ursprung eines Koordinatensystems bildet, und eine Patientenpositioniervorrichtung zum automatischen Positionieren des Patienten in Bezug zum eingestellten Behandlungsraum-Isozentrum aufweist.

## Beschreibung

Die Erfindung betrifft eine Partikeltherapieanlage zum Bestrahlen eines zu bestrahlenden Volumens eines Patienten mit hochenergetischen Partikeln. Die Partikeltherapieanlage weist mindestens einen Behandlungsraum mit einem Strahlaustritt, aus dem ein Partikelstrahl austritt, um mit dem in einer Bestrahlungsposition positionierten Patienten wechselzuwirken. Üblicherweise ist die Bestrahlungsposition in Bezug zu einem Bestrahlungsisozentrum der Partikeltherapieanlage gegeben; die Partikeltherapieanlage weist üblicherweise ferner eine Bildgebungsvorrichtung zur Verifikation der Lage des Zielvolumens bezüglich des Partikelstrahls und eine Patientenpositioniervorrichtung, mit der der Patient zur Bestrahlung in die Bestrahlungsposition bringbar ist, auf. Ferner betrifft die Erfindung die Planung und Durchführung einer Bestrahlung in einem Behandlungsraum einer derartigen Anlage.

Verschiedene Bestrahlungsanlagen und -techniken sind von H. Blattmann in "Beam delivery systems for charged particles", Radiat. Environ. Biophys. (1992) 31:219-231 beschrieben. Eine Partikeltherapieanlage ist beispielsweise aus EP 0 986 070 bekannt.

Eine Partikeltherapieanlage weist üblicherweise eine Beschleunigereinheit und ein Hochenergiestrahlführungssystem auf. Die Beschleunigung der Partikel, z.B. Protonen, Pionen, Helium-, Kohlenstoff- oder Sauerstoff-Ionen, erfolgt beispielsweise mit Hilfe eines Synchrotrons oder Zyklotrons.

Das Hochenergiestrahltransportsystem führt die Partikel von der Beschleunigereinheit zu einem oder mehreren Behandlungsräumen. Man unterscheidet zwischen "fixed beam" Behandlungsräumen, in denen die Partikel aus einer festen Richtung auf den Behandlungsplatz treffen, und so genannten Gantry basierten Behandlungsräumen. Bei letzteren ist es möglich, den Partikelstrahl aus verschiedenen Richtungen auf den Patienten zu richten.

Ein Kontroll- und Sicherheitssystem der Partikeltherapieanlage gewährleistet, dass jeweils ein mit den erbetenen Parametern charakterisierter Partikelstrahl in den entsprechenden Behandlungsraum geführt wird. Die Parameter werden im so genannten Behandlungs- oder Therapieplan definiert. Dieser gibt an, wie viele Teilchen aus welcher Richtung mit welcher Energie auf den Patienten treffen sollen.

Üblicherweise wird der Therapieplan mit Hilfe von bildgebenden Verfahren erzeugt. Dazu wird beispielsweise ein 3D-Datensatz mit einem Computertomographiegerät erzeugt. Der Tumor wird innerhalb dieses Bilddatensatzes lokalisiert und es werden die benötigten Strahlungsdosen, Einfallsrichtungen, Partikelsorten etc. festgelegt.

Bei der Bestrahlung ist es notwendig, dass der Patient die der Therapieplanung zu Grunde liegende Bestrahlungsposition einnimmt. Dies erfolgt beispielsweise mit fixierenden Masken. Zusätzlich wird vor der Bestrahlung die Position des Patienten mit bildgebenden Mitteln überprüft. Dabei findet ein Abgleich der aktuellen Bestrahlungsposition mit dem der Therapieplanung zugrunde liegenden Bilddatensatzes statt.

Bei dieser so genannten Positionsverifikation werden vor einer Bestrahlung Aufnahmen aus verschiedenen Richtungen mit beispielsweise Projektionen aus dem CT-Planungsdatensatz abgeglichen. Dazu werden u.a. auch Durchleuchtungsaufnahmen in Strahlrichtung und orthogonal zur Strahlrichtung gewonnen. Diese Aufnahmen werden in der Bestrahlungsposition nahe dem Strahlaustritt durchgeführt; d.h., es steht nur eingeschränkt Platz für die Bildgebung zur Verfügung.

Allgemein gibt es Bildgebungsverfahren zur Gewinnung von 3D-Bilddatensätzen, die darauf beruhen, dass Durchleuchtungen aus verschiedenen Richtungen durchgeführt werden. Aus den Bilddaten können ähnlich zu einer CT-Aufnahme 3D-artige Bilddatensätze gewonnen werden. Eine Möglichkeit für eine derartige Bildgebungsvorrichtung ist ein Imaging-Roboter, der frei um einen zu durchleuchtenden Patienten ausgerichtet werden kann. Zur Durchleuchtung des Patienten aus verschiedenen Richtungen muss entsprechend ausreichend Platz zur Verfügung stehen. Eine andere Möglichkeit zur Gewinnung von 3D-Aufnahmen ist z.B. ein C-Arm-Röntgenapparat.

Derartige Bildgebungseinheiten zur Gewinnung von 3D-Bilddatensätzen benötigen ausreichend Platz, um den Patienten von verschiedenen Richtungen durchleuchten zu können. D.h., Elemente der Bildgebungseinheit müssen zur Bildgebung in ausreichendem Abstand um den Patienten bewegt werden können.

Allgemein ist es bei einer Partikeltherapie von Vorteil, den Patienten so nah am Strahlaustritt zu positionieren, um die Aufweitung des Strahls durch Streuung so gering wie möglich zu halten. Ein üblicher Abstand zwischen dem Isozentrum eines Bestrahlungsplatzes und dem Strahlaustritt liegt bei ca. 60 cm.

Der oben angesprochene bevorzugte Abstand des Bestrahlungsisozentrums vom Strahlaustritt schränkt die Bildgebung der Positionsverifikation auf entsprechend wenig Platz einnehmende Bildgebungsvorrichtungen ein.

Eine Aufgabe der Erfindung ist es, die Planung und Durchführung einer Bestrahlung eines Patienten so zu ermöglichen, dass die Leistungsfähigkeit einer flexibel einsetzbaren und hochpräzisen Therapieanlage ausgenutzt wird. Ferner ist es eine Aufgabe der Erfindung, die Partikeltherapie mit verschiedenen Partikelsorten, mittels Scan-Technik und mit einer hochgenauen Positionsverifikation möglich zu machen. So ist es z.B. eine Aufgabe der Erfindung, ein Verfahren und eine Partikeltherapieanlage anzugeben, die es erlauben, auch platzbeanspruchende Bildgebungstechniken bei der Positionsverifikation zu verwenden.

Die Aufgabe wird durch einen Behandlungsplatz nach Anspruch 1 gelöst. Ferner wird die Aufgabe bezogen auf die Planung durch einen Therapieplan nach Anspruch 15 und durch ein Verfahren zur Erstellung eines Therapieplans nach Anspruch 16. Ferner wird die Aufgabe durch ein Bestrahlungsverfahren nach Anspruch 19 gelöst.

In einer Ausführungsform des Behandlungsraums weist dieser ein während einer Behandlung variabel einstellbares Behandlungsraum-Isozentrum, welches einen Ursprung eines Koordinatensystems bildet, und eine Patientenpositioniervorrichtung zum automatischen Positionieren des Patienten in Bezug zum eingestellten Behandlungsraum-Isozentrum auf.

Das Behandlungsraum-Isozentrum stellt den Ursprung eines Koordinatensystems im Behandlungsraum dar. In Bezug zu ihm werden Positionierungen z.B. von der Patientenlagerungsvorrichtung, vom Patienten, von einer Bildgebungseinheit und/oder von einem Partikelstrahlverlauf im Behandlungsraum definiert. Seine Lage entlang des Partikelstrahlverlaufs definiert die bei der Bestrahlung vorliegenden Strahlparameter wie Strahldurchmesser und Strahlprofil, insbesondere der Steilheit des Abfalls im Strahlprofil.

Ist das Behandlungsraum-Isozentrum einstellbar, bedeutet dies, dass das Behandlungsraum-Isozentrum nicht mehr auf einen Punkt im Behandlungsraum festgelegt ist, sondern dass es - evtl. eingeschränkt auf einen Bereich - frei gewählt und eingestellt werden kann. So kann das Behandlungsraum-Isozentrum z.B. durch ein entsprechend ausrichtbares Laserkreuz gezielt im Raum kenntlich gemacht werden. Zusätzlich oder alternativ ist es möglich, das Behandlungsraum-Isozentrum bzw. seine Position oder Koordination im Behandlungsraum als gespeicherte Information z.B. in einem Therapiekontrollzentrum abzulegen und bei der Steuerung eines Bestrahlungsvorgangs zu verwenden. Dazu wird es beispielsweise der Positioniervorrichtung übermittelt und/oder der Ansteuerung der Positioniervorrichtung zugrunde gelegt, wenn ein Therapieplan-Isozentrum in Bezug zum Behandlungsraum-Isozentrum zu setzen ist. Ferner kann es einer Bildgebungseinheit übermittelt und/oder der Ansteuerung der Bildgebungseinheit zugrunde gelegt werden, wenn eine Bildgebung z.B. zentriert um das Behandlungsraum-Isozentrum durchzuführen ist.

Da die Strahlparameter wie Strahldurchmesser und Strahlprofil, insbesondere der Steilheit des Abfalls im Strahlprofil, neben der Lage des Behandlungsraum-Isozentrums im Strahlverlauf auch von der Partikelsorte abhängen, ist es von besonderem Vorteil, wenn das Behandlungsraum-Isozentrum entsprechend flexibel einstellbar ist.

Somit liegt ein weiterer Vorteil der Erfindung darin, dass für jeden Bestrahlungsvorgang, d.h. u.a. für jede Partikelsorte und für jede Bestrahlungsrichtung, ein optimaler Abstand des Behandlungsraum-Isozentrums zu einem Strahlaustritt im Behandlungsraum eingestellt werden kann. Zusammen mit einem entsprechend einstellbaren kleinen Strahldurchmesser weist ein derartiger Strahl dann z.B. zusätzlich einen steilen radialen Abfall in der Partikelverteilung auf. Dadurch kann eine hochgenaue und präzise Bestrahlung, wie sie z.B. mit einer Rasterscan-Technik möglich ist, optimal umgesetzt werden.

Des Weiteren kann bei entsprechender Wahl des Behandlungsraum-Isozentrums eine 3D-Bildgebung auch mit einer entsprechend platzbeanspruchenden Bildgebungsvorrichtung erfolgen. Dadurch kann hinsichtlich der Positionsverifikation eine sehr präzise Bestrahlung mit einem Partikelstrahl durchgeführt werden, da die Positionsverifikation mit 3D- oder zumindest 3D-artigen Datensätzen erfolgt.

In einer vorteilhaften Ausführungsform des Behandlungsraumes ist das einstellbare Behandlungsraum-Isozentrum entlang einer Strahlmittenachse des Partikelstrahls insbesondere für Bestrahlungsvorgänge einstellbar. Dabei ist unter der Strahlmittenachse beispielsweise der durch die Nullposition einer Rasterscanvorrichtung gegebene Strahlverlauf zu verstehen.

In einer weiteren vorteilhaften Ausführungsform beträgt die Entfernung zwischen Behandlungsraum-Isozentren zur Bestrahlung und zur Bildgebung nicht mehr als 2 m und wenn möglich weniger als 0,5 m, so dass während einer Bestrahlung die Positionsverifikation wenn möglich ohne großen Zeitverlust durch lange Verfahrwege auch mehrfach vorgenommen werden kann. Dies ist insbesondere dann möglich, wenn der Verschiebeweg des Patienten so gering wie möglich gehalten wird, d.h., wenn z.B. die Bildgebungseinheit den bzw. nahezu den Mindestabstand zum Strahlaustritt aufweist.

In einer vorteilhaften Ausführungsform umfasst die Patientenpositioniervorrichtung einen robotisch angesteuerten Patiententisch. Bevorzugt wird die Patientenpositioniervorrichtung von einer Therapiekontrolleinheit der Partikeltherapieanlage angesteuert. Dies hat den Vorteil, dass beispielsweise die Parameter zur Durchführung eines Positionswechsels im Therapieplan abgelegt werden können, welcher der Therapiekontrolleinheit zur Steuerung der Bestrahlung zugrunde liegt.

Vorzugsweise umfasst eine Ausführungsform eines erfindungsgemäßen Therapieplans mindestens zwei Vorgänge zur Bestrahlung und/oder Bildgebung mit identischen und/oder unterschiedlichen Therapieplan-Isozentren, wobei die Vorgänge mindestens zwei räumlich verschieden Behandlungsraum-Isozentren zugeordnet sind. Unter einem Therapieplan-Isozentrum ist dabei ein Punkt (Volumenelement) in einem der Planung zugrunde liegenden Bilddatensatz des zu bestrahlenden Patienten zu verstehen. In Bezug zu diesem Punkt wird z.B. ein Bestrahlungsvorgang geplant, d.h., geometrische Information für die Bestrahlung, wie Bestrahlungsrichtung oder zu bestrahlendes/abzubildendes Volumen etc., wird auf diesen Punkt bezogen. Ferner wird ein bei der Durchführung des Vorgangs gewünschter Bezug des Therapieplan-Isozentrums zum Behandlungsraum-Isozentrum definiert. Bei der Planung werden auch gewünschte Strahlparameter, welche durch die Position des Behandlungsraum-Isozentrums gegeben sind, berücksichtigt.

Der Bezug des Therapieplan-Isozentrums zum Behandlungsraum-Isozentrum -z.B. ein aufeinander legen der beiden Isozentrenwird dann bei der Durchführung hergestellt, indem z.B. der Patient oder die Bildgebungsvorrichtung positioniert und/oder das Behandlungsraum-Isozentrum entsprechend eingestellt werden.

Ein derartiger Therapieplan hat den Vorteil, dass schon bei der Therapieplanung die Freiheit über die räumliche Wahl der zu verwendenden Behandlungsraum-Isozentren, d.h. z.B. deren Abstand zum Strahlaustritt, in eine optimierte Partikeltherapie eingebracht werden kann. Dabei kann z.B. die Positionsverifikation unabhängig von der Position eines für eine Bestrahlung eingestellten Behandlungsraum-Isozentrums erfolgen oder Behandlungsraum-Isozentren in Abhängigkeit der Einfallswinkel und der verwendeten Partikelsorten gewählt werden.

Weitere vorteilhafte Ausführungsformen der Erfindung sind durch die Merkmale der Unteransprüche gekennzeichnet.

Es folgt die Erläuterung von mehreren Ausführungsbeispielen der Erfindung anhand der Figuren 1 bis 5. Es zeigen:
- FIG 1: schematisch eine Ausführungsform einer Partikeltherapieanlage zur Verdeutlichung der Erfindung,
- FIG 2: ein beispielhaftes Flussdiagramm zur Verdeutlichung eines Bestrahlungsverfahrens nach der Erfindung in Wechselwirkung mit einem Therapieplan und
- FIG 3 bis 5: schematische Darstellungen eines Behandlungsraumes zur Verdeutlichung variabel einstellbarer Behandlungsraum-Isozentren.

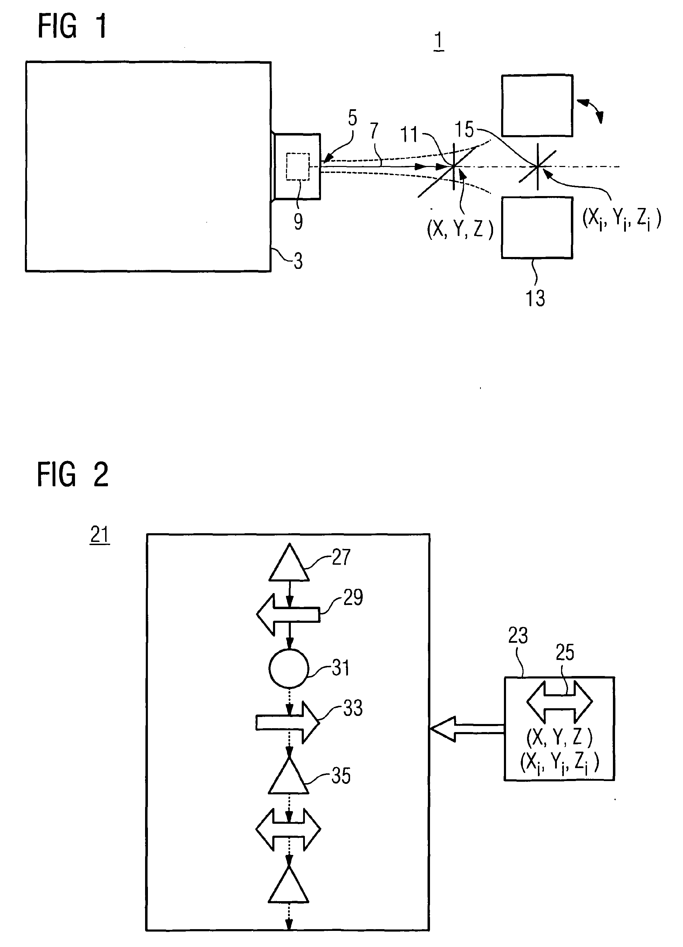

Figur 1 zeigt schematisch eine Partikeltherapieanlage 1 zum Bestrahlen eines zu bestrahlenden Volumens eines Patienten mit hochenergetischen Partikeln. Eine Partikelbeschleunigereinheit 3 emittiert dazu aus einem Strahlaustritt 5 einen Partikelstrahl 7. Umfasst die Partikeltherapieanlage beispielsweise eine Rasterscanvorrichtung 9, so kann ein Scanbereich von beispielsweise 20 cm x 20 cm abgescannt werden. Ein Behandlungsraum-Isozentrum 11 ist vorzugsweise auf einer Strahlmittenachse, die mittig zum Scanbereich verläuft, einstellbar. Der Partikelstrahl divergiert aufgrund von Streuvorgängen im Strahl oder mit durchstrahlter Materie. Je näher ein Behandlungsraum-Isozentrum am Strahlaustritt 5 angeordnet ist, desto kleiner ist der Strahldurchmesser der Partikelverteilung im Partikelstrahl und desto schärfer ist der seitliche Abfall der Partikelverteilung definiert. Bei der Bestrahlung mit Protonen wird vorzugsweise ein Abstand von 60 cm gewählt. In diesem Abstand divergiert der Strahl auf einen gewünschten und im Therapieplan angenommenen Strahldurchmesser; beispielsweise erfolgt die Bestrahlung mit einem Rasterscanverfahren mit einem Strahldurchmesser von ca. 5 mm.

Ferner weist die Partikeltherapieanlage 1 eine Bildgebungsvorrichtung 13 auf, die vorzugsweise auch zur Erzeugung eines 3D-Datensatzes des Patienten im Bereich des zu bestrahlenden Volumens ausgebildet ist. Mithilfe der Bildgebungsvorrichtung 13 soll die Positionsverifikation des zu bestrahlenden Volumens bezüglich des Partikelstrahls vorgenommen werden. Die Bildgebungsvorrichtung 13 weist ein Bildgebungszentrum 15 auf. Der Abstand des Bildgebungszentrums 15 vom Strahlaustritt 5 ist bedingt durch die Ausbildung, d.h. die Ausmaße und Struktur, der Bildgebungsvorrichtung 13 größer als der Abstand des zur Bestrahlung vorgesehenen Behandlungsraum-Isozentrums 11 vom Strahlaustritt 5. Zur Bildgebung wird ein Behandlungsraum-Isozentrum vorgesehen, das - wie in der Figur 1 durch das Bildgebungszentrum 15 angedeutet - ebenfalls auf der Strahlmittenachse angeordnet ist. Der Abstand zwischen dem Behandlungsraum-Isozentrum 11 und dem Behandlungsraum-Isozentrum für die Bildgebung (Bildgebungszentrum 15) wird möglichst klein gehalten, beispielsweise ist der Abstand des Bestrahlungszentrums 15 vom Strahlaustritt 5 100 cm. Eine Verschiebung in oder entgegen der Strahlrichtung von 40 cm kann schnell und ohne Belastung des Patienten auch während einer Bestrahlungssitzung durchgeführt werden.

Figur 2 verdeutlicht eine Bestrahlungssitzung 21, die auf Grundlage eines Therapieplans 23 durchgeführt wird. Der Therapieplan 23 weist neben den benötigten Strahlparametern die Partikelenergie, die Partikelintensität und Einfallsrichtung für beispielsweise verschiedene Volumenelemente des zu bestrahlenden Volumens und verschiedene Bestrahlungsvorgänge aus verschiedenen Richtungen auf. Zusätzlich enthält er Information über die Lage (X,Y,Z) der Behandlungsraum-Isozentren zur Bestrahlung und/oder die Lage (Xᵢ,Yᵢ,Zᵢ) der Behandlungsraum-Isozentren zur Bildgebung und/oder evtl. einen Verschiebevektor 25, der angibt um wie viel ein Patient oder eine Bildgebungsvorrichtung verschoben werden muss, damit Therapieplan-Isozentren mit Behandlungsraum-Isozentren abgeglichen sind.

Die Bestrahlungssitzung 21 beginnt vorzugsweise mit einer Positionsverifikation 27, bei der der Patient entsprechend der Therapieplanung im Behandlungsraum-Isozentren zur Bildgebung (Xᵢ, Yᵢ, Zᵢ) in der Bildgebungsposition positioniert ist. Anschließend wird eine Verschiebung 29 gemäß dem Verschiebungsvektor 25 durchgeführt. Nun befindet sich der Patient in der Bestrahlungsposition. In dieser Position wird ein erster Bestrahlungsvorgang 31 durchgeführt.

Entsteht während der Bestrahlung beispielsweise der Verdacht, dass sich die Position des Patienten geändert hat, kann nun eine zweite Verschiebung 33 zurück in die Bildgebungsposition erfolgen, um eine weitere Positionsverifikation 35 durchzuführen.

Derartige Positionsverifikationen können wiederholt auftreten sei es aufgrund von vermuteten Positionsänderungen, aus Sicherheitsgründen oder um eine weitere Bestrahlung beispielsweise aus einer anderen Einfallsrichtung vorzunehmen.

Die Erstellung des Therapieplans 23 für die Bestrahlungssitzung 21, die evtl. mehreren Bestrahlungs- und/oder Bildgebgungsvorgängen aufweist, erfolgt z.B. in mehreren Schritten. In einem Schritt wird ein Bildgebungsvorgang geplant, bei dem ein Therapieplan-Isozentrum des zu bestrahlenden Volumens im Bildgebungszentrum der Bildgebungsvorrichtung liegt. In dieser Position (der Bildgebungsposition) soll die Bildgebung zur Verifikation der Position des Patienten gemäß der Bestrahlungsplanung durchgeführt werden. Es wird kein Strahl in dieser Bildgebungsposition geplant und appliziert.

In einem anderen Schritt wird ein Bestrahlungsvorgang geplant. Dazu werden ein oder mehrere Behandlungsraum-Isozentren festgelegt und es werden ein oder mehrere Bestrahlungsfelder geplant. Die Planung des Bestrahlungsvorgangs umfasst z.B., dass zu Beginn des Bestrahlungsvorgangs der Patient mittels der Patientenpositioniervorrichtung so positioniert wird, dass das Bestrahlungsisozentrum in einem Isozentrum des Bestrahlungsplatzes liegt. Ein Behandlungsraum-Isozentrum wird dabei so geplant, dass der Patient ohne Gefährdung möglichst nahe an den Strahlaustritt heranfährt, d.h., das Behandlungsraum-Isozentrum wird vom Bildgebungszentrum zur für die Bestrahlung geplanten Position verschoben. In dieser Position (der Bestrahlungsposition) erfolgt dann die eigentliche Bestrahlung.

Weitere Bildgebungsvorgänge und Bestrahlungsvorgänge unter Umständen auch unter geänderten Einfallsrichtungen, können bei Bedarf mitgeplant werden. Dabei können die unterschiedliche Strahleinfallsrichtung bei Verwendung einer Gantry entsprechend angepasste Behandlungsraum-Isozentren bedingen.

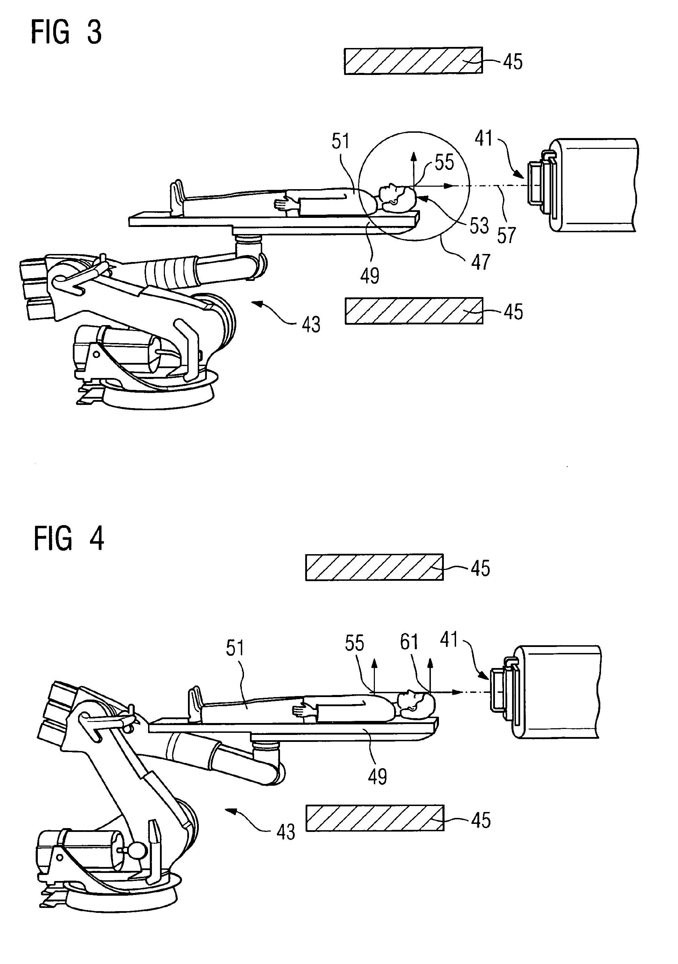

Figur 3 zeigt ein Beispiel eines Behandlungsraums mit einem Strahlaustritt 41, einer Patientenpositioniervorrichtung 43 und einer Bildgebungsvorrichtung 45 mit einem Bildgebungsvolumen 47. Die Patientenpositioniervorrichtung 43 weist eine Patientenliege 49 auf, auf der ein Patient 51 liegt. Das zu bestrahlende Volumen des Patienten 51 liegt beispielsweise innerhalb eines Schädels 53 des Patienten 51. Das Bildgebungsvolumen 47 weist ein Bildgebungszentrum 55 auf. Das Bildgebungszentrum 55 befindet sich bevorzugt auf einer Strahlmittenachse 57 des Partikelstrahls z.B. in einer Entfernung von 100 cm zum Strahlaustritt 41. Zur Positionsverifikation soll eine Aufnahme, bevorzugt eine 3D-Aufnahme des zu bestrahlenden Volumens mit Hilfe der Bildgebungsvorrichtung 45 aufgenommen. Dazu wird das Behandlungsraum-Isozentrum auf die im Therapieplan vorgesehene Position eingestellt. Die Einstellbarkeit des Behandlungsraum-Isozentrums erlaubt es, die Bildgebungsvorrichtung in allen zur 3D-Bildgebung benötigten Stellungen zu planen.

Die 3D-Aufnahme wird mit Aufnahmen, die der Therapieplanung zugrunde lagen, abgeglichen und falls nötig wird der Patient 51 mit Hilfe der Patientenpositioniervorrichtung 43 in die der Therapieplanung zu Grunde liegenden Position nachjustiert. Er befindet sich dann in der im Therapieplan definierten Bildgebungsposition.

Von der Bildgebungsposition wird der Patient 51 in die Bestrahlungsposition, welche in Figur 4 dargestellt ist, verschoben. Dazu wird das Behandlungsraum-Isozentrum auf die im Therapieplan vorgesehene Position eingestellt. Das zuvor um das Bildgebungszentrum 55 gelegene, zu bestrahlende Volumen liegt nun um das Bestrahlungsisozentrum 61 und kann beispielsweise mit Hilfe einer (Raster-)Scan-Vorrichtung volumenelementspezifisch bestrahlt werden.

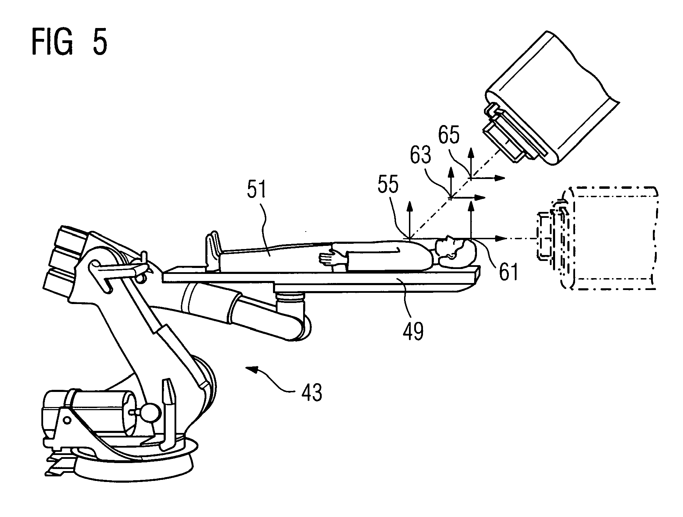

In Figur 5 wurde im Unterschied zur Figur 4 der Strahlaustritt als Teil einer Gantry angenommen und um einen Winkel in eine weitere Bestrahlungsposition mit einem anderen Einfallswinkel rotiert. Eine ähnliche Situation kann bei einem Behandlungsraum mit zwei Strahlaustrittsmöglichkeiten vorliegen. Für die Bestrahlung mit z.B. Protonen aus diesem Winkel ist ein Behandlungsraum-Isozentrum 63 und zur Bestrahlung mit Kohlenstoff-Ionen ist ein Behandlungsraum-Isozentrum 65 angedeutet. Die Behandlungsraum-Isozentren sind auf die Partikelarten im Abstand zum Strahlaustritt optimierbar. Umfasst der Therapieplan einen Bestrahlungsvorgang mit einer dieser Partikelarten unter diesem Winkel, wird der Patient zur Bestrahlung so verschoben, dass das zugehörige Therapieplan-Isozentrum mit dem Behandlungsraum-Isozentrum abgeglichen ist. Dazu wird die Bildgebungseinheit verfahren und die Positioniereinheit 43 entsprechend dem jeweiligen Behandlungsraum-Isozentrum angesteuert.

## Patentansprüche

1. Behandlungsraum für eine Partikeltherapieanlage aufweisend ein während einer Behandlung variabel einstellbares Behandlungsraum-Isozentrum, welches einen Ursprung eines Koordinatensystems bildet, und mit einer Patientenpositioniervorrichtung zum automatischen Positionieren des Patienten in Bezug zum eingestellten Behandlungsraum-Isozentrum.

2. Behandlungsraum nach Anspruch 1,
**dadurch gekennzeichnet , dass** das variable Behandlungsraum-Isozentrum auf einer Strahlmittenachse eines im Behandlungsraum verlaufenden Partikelstrahls variabel einstellbar ist.

3. Behandlungsraum nach Anspruch 2,
**dadurch gekennzeichnet, dass** mindestens ein Strahlparameter, wie eine Strahlbreite, ein Strahlprofil und/oder eine abfallende Flanke des Strahlprofils, mittels der Wahl der Position des variablen Behandlungsraum-Isozentrum auf der Strahlmittenachse für einen Bestrahlungsvorgang einstellbar ist.

4. Behandlungsraum nach einem der Ansprüche 1 bis 3,
**dadurch gekennzeichnet , dass** zusätzlich mindestens ein den Partikelstrahl charakterisierender Parameter wie Strahlfokus, Strahldivergenz und/oder Strahldurchmesser im Behandlungsraum einstellbar ist.

5. Behandlungsraum nach einem der Ansprüche 1 bis 4,
**dadurch gekennzeichnet , dass** die Patientenpositioniervorrichtung einen robotisch angesteuerten Patiententisch umfasst, der zur Patientenverschiebung von der Bildgebungsposition zur Bestrahlungsposition insbesondere von einer Therapiekontrolleinheit der Partikeltherapieanlage ansteuerbar ist.

6. Behandlungsraum nach einem der Ansprüche 1 bis 5,
**dadurch gekennzeichnet , dass** ein steuerbares Laserkreuz die eingestellte Position des variablen Behandlungsraum-Isozentrums im Behandlungsraum kennzeichnet.

7. Behandlungsraum nach einem der Ansprüche 1 bis 6,
**dadurch gekennzeichnet , dass** ein Strahlaustritt eines Strahlzuführungs- und Beschleunigungssystems vorhanden ist, aus dem ein Partikelstrahl austritt, um mit einem in einer Bestrahlungsposition positionierten Patienten wechselzuwirken, wobei die Bestrahlungsposition durch die Lage des eingestellten Behandlungsraum-Isozentrums gegeben ist.

8. Behandlungsraum nach einem der Ansprüche 1 bis 7,
**dadurch gekennzeichnet , dass** das Behandlungsraum-Isozentrum in seiner Entfernung zum Strahlaustritt insbesondere in Abhängigkeit der Partikelart, z.B. Protonen, Kohlenstoff- oder Sauerstoff-Ionen, einstellbar ist.

9. Behandlungsraum nach einem der Ansprüche 1 bis 8,
**dadurch gekennzeichnet , dass** eine Bildgebungsvorrichtung zur Verifikation der Lage des zu bestrahlenden Volumens bezüglich des Partikelstrahls vorhanden ist, die zur Verifikation der Lage des zu bestrahlenden Volumens in einer Bildgebungsposition des Patienten ausgebildet ist, wobei die Bildgebungsposition durch die Lage des eingestellten Behandlungsraum-Isozentrums gegeben und insbesondere von der Bestrahlungsposition räumlich entfernt angeordnet ist.

10. Behandlungsraum nach Anspruch 9,
**dadurch gekennzeichnet , dass** der Bildgebungsposition ein Bildgebungszentrum zuordenbar ist, das insbesondere ebenso wie das Bestrahlungsisozentrum auf der Strahlmittenachse angeordnet ist.

11. Behandlungsraum nach einem der Ansprüche 9 oder 10,
**dadurch gekennzeichnet , dass** die Bildgebungsvorrichtung zur 3D-Bildgebung ausgebildet ist.

12. Behandlungsraum nach einem der Ansprüche 9 bis 11,
**dadurch gekennzeichnet , dass** ein für die Bildgebungseinheit zur Verfügung stehender Raum mittels einer Einstellung des variablen Behandlungsraum-Isozentrum auf der Strahlmittenachse einstellbar ist.

13. Behandlungsraum nach einem der Ansprüche 9 bis 12,
**dadurch gekennzeichnet , dass** die Bildgebungsvorrichtung Abmessungen aufweist, die einen Mindestabstand zum Strahlaustritt definieren, und dass die Bildgebungsvorrichtung mindestens in diesem Mindestabstand vom Strahlaustritt angeordnet ist, wobei der Mindestabstand größer ist als die Entfernung zwischen Strahlaustritt und Bestrahlungsisozentrum.

14. Behandlungsraum nach einem der Ansprüche 9 bis 13,
**dadurch gekennzeichnet , dass** die Bildgebungsvorrichtung ein C-Bogen-Röntgengerät oder ein Imaging-Roboter ist, welche für eine 3D-Bildgebung rotierbar um die Bildgebungsposition, insbesondere um das Bildgebungszentrum, ausgebildet sind, und dass ein Mindestabstand zum Strahlaustritt durch die Rotierbarkeit bestimmt ist und die Bildgebungsvorrichtung mindestens in diesem Mindestabstand vom Strahlaustritt angeordnet ist.

15. Therapieplan zum Bestrahlen eines Patienten in einem Behandlungsraum mit Partikeln einer Partikeltherapieanlage, aufweisend
- mindestens zwei Vorgänge zur Bestrahlung und/oder Bildgebung mit identischen und/oder unterschiedlichen Therapieplan-Isozentren, wobei die Vorgänge mindestens zwei räumlich verschieden Behandlungsraum-Isozentren zugeordnet sind.

16. Verfahren zur Erstellung eines Therapieplans nach Anspruch 15, wobei zur Bestimmung einer Strahlendosisverteilung und Optimierung eines Bestrahlungsvorgangs, eine Datenbasis verwendet wird, in der charakteristische Strahlparameter für verschiedenen Behandlungsraum-Isozentren, insbesondere in Abhängigkeit des Abstandes des Behandlungsraum-Isozentrums von einem Strahlaustritt oder Strahlfokus, abgelegt sind.

17. Verfahren nach Anspruch 16, wobei im Therapieplan Bestrahlungsvorgängen aus unterschiedlichen Bestrahlungsrichtungen und/oder mit unterschiedlichen Partikelsorten verschiedene Behandlungsraum-Isozentren zugeordnet werden.

18. Verfahren nach Anspruch 16 oder 17, wobei der Bestrahlungsvorgang für eine Partikelbestrahlüng, die eine Scan-Technik, insbesondere eine Rasterscan-Technik benutzt, geplant wird.

19. Bestrahlungsverfahren zum Bestrahlen eines zu bestrahlenden Volumens eines Patienten mit hochenergetischen Partikeln einer Therapieanlage mit mindestens zwei Vorgänge zur Bestrahlung und/oder Bildgebung mit identischen und/oder unterschiedlichen Therapieplan-Isozentren in einem Behandlungsraum,
- wobei den Therapieplan-Isozentren von mindestens zweien der Vorgänge räumlich verschiedene Behandlungsraum-Isozentren zugeordnet sind,
- wobei zwischen Vorgängen, denen räumlich verschiedene Behandlungsraum-Isozentren zugeordnet sind, das Behandlungsraum-Isozentrum umgestellt wird und
- wobei der Patient zur Durchführung der Vorgänge jeweils derart positioniert wird, dass das jeweilig geplanten Therapieplan-Isozentren und das jeweils eingestellten Behandlungsraum-Isozentrum abgeglichen sind.

20. Bestrahlungsverfahren nach Anspruch 19, wobei Bestrahlungsvorgängen aus unterschiedlichen Bestrahlungsrichtungen und/oder mit unterschiedlichen Partikelsorten verschiedene Behandlungsraum-Isozentren zugeordnet sind.

21. Bestrahlungsverfahren nach Anspruch 19 oder 20, wobei in mindestens einem Positionsänderungsvorgang ein Positionswechsel des Patienten zwischen zwei räumlich unterschiedlichen Behandlungsraum-Isozentren mittels Ansteuerung einer Patientenpositioniereinheit erfolgt, insbesondere wobei zum Positionswechsel des Positionsänderungsvorgangs eine Verschiebung des Patienten in oder entgegen der Bestrahlungsrichtung erfolgt.
